# EUROPÄISCHE PATENTSCHRIFT

(11) **EP 2 470 695 B1**
(45) Veröffentlichungstag und Bekanntmachung des Hinweises auf die Patenterteilung: **23.10.2013**
(21) Anmeldenummer: 10757382.6
(22) Anmeldetag: 20.07.2010
(51) Int. Cl.: D01F 2/28

(54) **CARBOXYETHYLCELLULOSEFASERN, IHRE VERWENDUNG IN WUNDVERBÄNDEN UND HYGIENEARTIKELN SOWIE VERFAHREN ZU IHRER HERSTELLUNG**
CARBOXYETHYL CELLULOSE FIBERS, THEIR USE IN WOUND DRESSINGS AND HYGIENE ITEMS AND METHOD FOR PRODUCING THE SAME
FIBRES EN CARBOXYÉTHYLCELLULOSE, LEUR UTILISATION DANS DES PANSEMENTS ET ARTICLES D'HYGIÈNE ET LEUR PROCÉDÉ DE FABRICATION

(30) Priorität: 28.08.2009 AT 13572009
(43) Veröffentlichungstag der Anmeldung: 04.07.2012
(73) Patentinhaber: Lenzing AG, 4860 Lenzing (AT)
(72) Erfinder: KRAJCIK, Rastislav, A-4840 Vöcklabruck (AT); RAHBARAN, Shayda, A-4840 Vöcklabruck (AT)
(74) Vertreter: Hanemann, Otto
(86) Internationale Anmeldenummer: PCT/AT2010/000265
(87) Internationale Veröffentlichungsnummer: WO 2011/022740

(56) Entgegenhaltungen:
- WO-A1-97/16595
- WO-A1-97/39170
- JP-A- 3 268 752

## Beschreibung

Die vorliegende Erfindung betrifft Carboxyethylcellulosefasern, ein Verfahren zu ihrer Herstellung sowie ihre Verwendung zur Wundversorgung, insbesondere in Wundverbänden, in anderen Produkten für medizinische Anwendungen wie beispielsweise Tupfern, Bandagen und Ähnlichem sowie in Hygieneartikeln, und in all diesen Verwendungen insbesondere zur Herstellung der Kontaktfläche zum Körper. Die aus den erfindungsgemäßen Fasern hergestellten Produkte kleben trotz außerordentlich hoher Saugfähigkeit nicht auf den Wunden bzw. auf der Haut und weisen einen so guten Zusammenhalt im gequollenen Zustand auf, dass sich insbesondere die daraus hergestellten Wundverbände sich in einem Stück wieder von der Wunde abziehen lassen, ohne diese zu verletzen.

### Stand der Technik

Fasern aus Cellulose sind für die Anwendung zur Versorgung von Wunden und auch im Hygienebereich grundsätzlich sehr gut geeignet, da die Cellulose eine gute Verträglichkeit mit der Haut und auch mit Wunden aufweist. Neben natürlichen Cellulosefasern wie Baumwolle sind seit langem auch cellulosische Kunstfasern wie Viskose, Lyocell, Cupro oder Polynosic kommerziell erhältlich und für diese Anwendungsbereiche bekannt. Lyocell ist gemäß der BISFA-Definition eine aus einem organischen Lösungsmittel gesponnene Faser. Mögliche Herstellungsverfahren hierfür sind unter anderem in der US 4246221 und der US 4196282 beschrieben. Herstellverfahren für die übrigen genannten cellulosischen Kunstfasern sind bereits viel länger bekannt.

Die Verwendung saugfähiger cellulosischer Materialien in medizinischen Anwendungen, beispielsweise in Wundverbänden ist seit langem bekannt, unter anderem aus US 4203435.

Die AT 363578 beschreibt die Herstellung saugfähiger cellulosebasierter Fasern durch Einspinnen von Carboxymethylcellulose und anderen Cellulosederivaten in Viskose.

Ebenfalls finden chemisch modifizierte Polysaccharide Verwendung als saugfähige Bestandteile von Wundverbänden und -auflagen, beispielsweise gemäß EP 0092999 in Form wasserdispergierbarer Hydrokolloide aus Carboxymethylcellulose oder gemäß EP 0680344 in Form von Cellulosefasern, die nach dem Ausspinnen aus einer NMMO-Lösung carboxymethyliert wurden.

Wundverbände, die Carboxymethylcellulosefasern enthalten, haben jedoch den Nachteil, dass die Derivatisierung der Fasern unter Einsatz von Monochloressigsäure erfolgt, was zu einer Verringerung der Festigkeit der Fasern und damit später zu einem unzureichenden Zusammenhalt der durch Flüssigkeitsaufnahme gequollenen Gelschicht führt.

Verfahren zur Herstellung von Carboxyethylcellulose werden im Stand der Technik ebenfalls bereits beschrieben. So schlägt beispielsweise die US20060137838 die Herstellung von Carboxyethylcellulose aus Zellstoff in gleicher Weise wie von Carboxymethylcellulose unter Verwendung der entsprechenden Chloralkylsäuren als Reagens vor. Dieses Verfahren ist zur Carboxymethylcellulose-Herstellung gut geeignet. Jedoch ist die analoge Herstellung von Carboxyethylcellulose-Fasern auf diese Art wirtschaftlich nicht möglich. Durch das Nacharbeiten dieser Vorschriften konnte keine wirtschaftlich relevante Ausbeute an Carboxyethylcellulose-Fasern erhalten werden. Die Fasern hatten kein signifikant höheres Wasserrückhaltevermögen im Vergleich zu underivatisierten Kontrollfasern. Es ist daher zu vermuten, dass die jeweiligen Angaben zur Carboxyethylcellulose-Herstellung lediglich theoretischer Natur sind und nicht technisch geprüft wurden.

Die US 5667637 offenbart die Herstellung von Carboxyethylcellulose ausgehend von Zellstoff mit Acrylamid für Papieranwendungen.

Die Verwendung von Carboxyethylcellulose wird in der Literatur auch häufig für Wundverbände vorgeschlagen, jedoch immer nur in Aufzählungen von verschiedenen theoretisch denkbaren Alternativen zu Carboxymethylcellulose. Praxisrelevante Eigenschaften solcher Carboxyethylcellulosen oder konkrete Beispiele dazu werden nicht aufgeführt. Auch Angaben über die Festigkeit solcher Fasern sind nicht zu finden.

### Aufgabe

Angesichts des Standes der Technik bestand die Aufgabe, ein alternatives cellulosebasiertes Material zur Absorption von Flüssigkeiten und insbesondere Körperflüssigkeiten, beispielsweise zur Verwendung in Wundverbänden und anderen Produkten für medizinische Anwendungen oder Hygieneanwendungen und darin insbesondere zur Herstellung der Kontaktfläche zum Körper sowie ein Verfahren zu seiner Herstellung zur Verfügung zu stellen. Dieses Material muß im gequollenen Zustand einen stärkeren Zusammenhalt als bisher bekannte Materialien aufweisen, die es beispielsweise ermöglichen, dass ein aus ihnen hergestellter Wundverband wieder im Ganzen von der Wunde abgezogen werden kann.

### Beschreibung der Erfindung

Diese Aufgabe konnte gelöst werden durch die erstmalige Bereitstellung von wasserunlöslichen Carboxyethylcellulosefasern, die eine Festigkeit im konditionierten Zustand von mindestens 15 cN/tex und unter Beibehaltung ihrer Faserform ein Wasserrückhaltevermögen von mindestens 400 % aufweisen.

Die Carboxyethylcellulosefasern weisen bevorzugt ein Wasserrückhaltevermögen von mindestens 600 %, besonders bevorzugt mindestens 800 % auf.

Die Festigkeit dieser Carboxyethylcellulosefasern im konditionierten Zustand beträgt bevorzugt mindestens 20 cN/tex. Prinzipiell sind Festigkeiten bis hin zu denen der underivatisierten Fasern möglich, also bis zu etwa 40 cN/tex. Überraschend wurde gefunden, dass die für die genannten Anwendungsgebiete geeigneten Carboxyethylcellulosefasern ausreichende mechanische Eigenschaften dann aufweisen, wenn sie durch Derivatisierung von Lyocell-, Viskose- oder Modalfasem hergestellt wurden. Diese mechanischen Eigenschaften ermöglichen es beispielsweise, dass ein unter Verwendung der erfindungsgemäßen Fasern hergestellter Wundverband nach Kontakt mit Wasser oder Wundflüssigkeit ein transparentes Gel bildet, dabei jedoch weiterhin eine hohe Festigkeit behält, die es ihm erlaubt, ohne Rückstände wieder von einer Wunde abgelöst zu werden. Auch für Hygieneartikel ist es von großer Bedeutung, dass die einsetzten Fasern nach erfolgter Absorption von Körperflüssigkeiten und die damit verbundene Quellung zum Gel weiterhin einen ausreichenden mechanischen Zusammenhalt aufweisen.

Da, wie bereits oben ausgeführt, die im Stand der Technik vorgeschlagenen Verfahren zur Herstellung von Carboxyethylcellulosefasern praktisch nicht zum Erfolg führen, bestand zunächst die Notwendigkeit, ein geeignetes Herstellungsverfahren zu entwickeln.

Gegenstand der vorliegenden Erfindung ist daher auch ein Verfahren zur Herstellung dieser wasserunlöslichen Carboxyethylcellulosefasern, in dem cellulosische Kunstfasern mit Acrylamid in einer starken Alkalilauge umgesetzt werden, wobei als cellulosische Kunstfasern Lyocell-, Viskose- oder Modalfasern eingesetzt werden, die Fasern nach der Umsetzung in einem nachfolgenden Schritt nochmals mit Alkalilauge nachbehandelt werden und zumindest in einem Schritt des Verfahrens die Reaktionslösung 1 bis 75 Gew.-% Ethanol enthält.

Der Titer der eingesetzten Fasern ist grundsätzlich frei wählbar und wird vom Anwendungszweck bestimmt. Für viele Anwendungen wird man aber eine nicht zu grobe Struktur der dem Körper zugewandten Fläche bevorzugen. Bevorzugt wird daher ein Einzelfasertiter von 0,5 dtex - 6,0 dtex, besonders bevorzugt 1,4 bis 3,3 dtex. Fasern mit einem Einzelfasertiter von kleiner als 0,5 dtex sind praktisch nicht relevant.

Die cellulosischen Kunstfasern können in Form von geschnittenen Einzelfasern - auch als Stapelfasern bezeichnet -, Filamenten, Endlosfaserkabel, Vliesstoffen, Geweben, Gestricken, Gewirken und/oder anderen textilen Flächengebilden eingesetzt werden.

Bevorzugte Alkalilauge ist Natronlauge. Allerdings ist der Einsatz jeder starken Lauge möglich. Die Laugenkonzentration sollte 2-10% betragen, bevorzugt jedoch 4-6%. Überraschenderweise wurde gefunden, dass die wässrige Lösung 1 bis 75% Ethanol enthalten kann, bevorzugt 15 bis 30 % Ethanol.

Die Menge an eingesetztem Acrylamid hängt eng mit dem gewünschten Substitutionsgrad zusammen. Pro Einheit Anhydroglucose können 2-10 Moleküle Acrylamid in der Reaktion eingesetzt werden. Bevorzugt werden 6-10 Moleküle Acrylamid pro Anhydroglucose verwendet und besonders bevorzugt 7 oder 8 Moleküle Acrylamid. Die Reaktion läuft 30-120 min., bevorzugt 50-70 min., bei einer Temperatur von 30-90°C, bevorzugt bei 40-60°C. Zusätztich kann nach dieser Reaktion die Reaktionstemperatur bis auf 90°C erhöht werden und weitere 30-120 min. behandelt werden. Bevorzugt wird eine Erhöhung um 10-40°C. Besonders bevorzugt wird die Temperatur auf 60-80°C erhöht und weitere 50-70 min. reagieren lassen. Die dabei erzielten Werte an Wasserrückhaltevermögen in 0,9% NaCl-Lösung erreichen 200-600%.

Diese Werte werden überraschenderweise deutlich gesteigert durch eine Nachbehandlung der Fasern mit einer 3-10%iger Alkalilauge, bevorzugt einer 4-6%igen Lauge. Als Lauge wird bevorzugt Natronlauge verwendet, aber prinzipiell ist jegliche Lösung eines Alkalimetallhydroxids geeignet. Die wässrige Laugenlösung kann 1 bis 75% Ethanol enthalten, bevorzugt 30 bis 70 %. Diese Nachbehandlung dauert 30-120 min., bevorzugt 50-70 min. bei einer Temperatur von 30-90°C, bevorzugt bei 60-80°C.

Wird im Reaktions- und/oder im Nachbehandlungsschritt kein Ethanol zugegeben, so weist das Endprodukt ein wesentlich geringeres Wasserrückhaltevermögen auf, als wenn erfindungsgemäß vorgegangen wird.

Die CEC Fasern werden nach der Nachbehandlung gewaschen und getrocknet. Zum Waschen wird einem Gemisch aus Ethanol, Wasser und einer schwachen Säure verwendet. Bevorzugt wird eine Lösung aus 20 - 80% Ethanol, 19 - 79% Wasser und 1-10% einer schwachen Säure, bevorzugt 40 - 70% Ethanol; 29 - 59% Wasser und 1-10% einer schwachen Säure. Als schwache Säure wird vorzugsweise Zitronen- oder Essigsäure verwendet. Zuletzt werden die Fasern mit einer Lösung aus einem Fettsäureester in Ethanol, bevorzugt Polyoxyethylen-Sorbitan-Fettsäureester, beispielsweise 1 % Tween 20, in Ethanol gewaschen und anschließend getrocknet.

Die finalen Fasern haben einen neutralen bis schwach sauren pH-Wert (pH 5,5-7,5) und ein Wasserrückhattevermögen von 400% bis über 1200% in 0,9%iger Kochsalzlösung.

Die so hergestellten Fasern können vor allem zur Herstellung von Produkten zur Absorption von Flüssigkeiten und Körperflüssigkeiten, beispielsweise zur Verwendung in Produkten für medizinische Anwendungen oder Hygieneanwendungen verwendet werden. Diese Fasern können auch für Produkte zum Feuchthalten von Wunden, z. B. mit Kochsalzlösung, verwendet werden. Die Verwendung erfolgt dabei in der Kontaktfläche, die dem Körper zugewandt bzw. mit ihm in Berührung ist. Im Falle von Wundverbänden, Pflastern, Bandagen, Tupfern und ähnlichem ist das üblicherweise die zu versorgende Wunde oder sonstige offene Stelle des Körpers. Im Falle von Hygieneartikeln, beispielsweise Babywindeln oder Inkontinenzprodukten und Damenhygieneartikel ist es eine dem Anwendungszweck entsprechende Hautoberfläche bzw. Körperstelle. Lediglich in Hygieneartikeln wird zwischen Haut und Faserschicht meist noch eine Schicht aus PP oder PES, ein sogenanntes top sheet eingesetzt. Dabei hat es sich für eine erfolgreiche Anwendung überraschenderweise als entscheidend herausgestellt, dass die Carboxyethylcellulosefasern eine Festigkeit im konditionierten Zustand von mindestens 15 cN/tex, bevorzugt mindestens 20 cN/tex und ein Wasserrückhaltevermögen von mindestens 400 % aufweisen. Nach derzeitigem Stand der Technik ist hierfür nur das oben beschriebene erfindungsgemäße Verfahren geeignet.

Die Carboxyethylcellulosefasern können vorzugsweise in Form von geschnittenen Einzelfasern - auch als Stapelfasern bezeichnet -, Filamenten, Endlosfaserkabel, Vliesstoffen, Geweben, Gestricken, Gewirken und/oder anderen textilen Flächengebilden eingesetzt werden.

Für die Verwendung in Wundverbänden kann die Carboxyethylcellulosefaser selbst oder auch ein anderer Teil des Wundverbandes mit Zusätzen von Ag-, Cu- und Zn-Verbindungen, Chitosan sowie mit anderen antimikrobiellen Komponenten versehen werden.

### Beispiele:

Die Erfindung soll nun anhand von Beispielen erläutert werden. Diese sind als mögliche Ausführungsformen der Erfindung zu verstehen. Keineswegs ist die Erfindung auf den Umfang dieser Beispiele eingeschränkt.

Bestimmung des Wasserrückhaltevermögens (anlehnt an Norm DIN 53814): Das Wasserrückhaltevermögen als Maß für die Saugfähigkeit der erfindungsgemäßen Fasern ist definiert als die Flüssigkeitsaufnahme durch Quellung einer bestimmten Menge Faser in Prozenten des Trockengewichtes und wird wie folgt bestimmt: 0,5 g Fasern werden in einem Schleudergefäß mit soviel 0,9%iger Kochsalzlösung versetzt, bis Flüssigkeit unten ausläuft. Danach wird noch mal mit Kochsalzlösung aufgefüllt und 2 Stunden stehen gelassen. Die Schleudergefäße werden anschließend 20 min. bei 3400 U/min (= 9500 m/s²) zentrifugiert und die Fasern danach in Wägegläsern gewogen. Danach werden die Fasern 16-18 Stunden bei 105°C getrocknet und nach dem Abkühlen wieder gewogen (gemäß. Die Differenz der beiden Massen multipliziert mit 100 und dividiert durch das Trockengewicht ergibt das Wasserrückhaltevermögen in Prozent. Die CEC Fasern erreichen Werte an Wasserrückhaltevermögen von mindestens 400%, bevorzugt mindestens 600% und besonders bevorzugt mindestens 800% in 0,9%iger Kochsalzlösung.

### Beispiel 1:

Lyocellfasern mit einem Einzelfasertiter von 1.4 dtex werden in eine 5,6%ige wässrige NaOH-Lösung gegeben. Die Lösung enthält weiter 25% Ethanol und 240g/l Acrylamid. Das Gemisch wird auf 50°C erhitzt und 60 min. reagieren lassen. Danach wird die Temperatur auf 70°C erhöht und weitere 60 min. reagieren lassen. Nach der Reaktion werden die Fasern mit einer Presswalze abgepresst auf eine Feuchtigkeit von 100%. Die abgepressten Fasern werden in 4%iger wässrige NaOH-Lösung, die 50% Ethanol enthält, 60 min. bei 70°C behandelt. Nach dieser zweiten Behandlung werden die Fasern wieder abgepresst und mit einer Lösung aus 55% Ethanol, 42% Wasser und 3% Zitronensäure gewaschen. Als letzter Behandlungsschritt folgt einmal Waschen mit 1 % Tween® 20 in Ethanol. Danach werden die Fasern getrocknet. Die so erhaltenen Fasern haben ein Wasserrückhaltevermögen von 900% in 0,9%iger NaCl-Lösung und eine Festigkeit konditioniert von 22 cN/tex.

### Beispiel 2 (Vergleichsbeispiel):

Um den Einfluß der Zugabe von Ethanol während der Reaktion und dem Nachbehandlungsschritt deutlich zu zeigern, wurde der Ethanolzusatz im folgenden Versuch weggelassen. Alles andere wurde gegenüber Beispiel 1 unverändert wiederholt.

Lyocellfasern mit einem Einzelfasertiter von 1.4 dtex werden in eine 5,6%ige wässrige NaOH-Lösung gegeben. Die Lösung enthält weiter und 240g/l Acrylamid. Das Gemisch wird auf 50°C erhitzt und 60 min. reagieren lassen. Danach wird die Temperatur auf 70°C erhöht und weitere 60 min. reagieren lassen. Nach der Reaktion werden die Fasern mit einer Presswalze abgepresst auf eine Feuchtigkeit von 100%. Die abgepressten Fasern werden in 4%iger wässrige NaOH-Lösung, 60 min. bei 70°C behandelt. Nach dieser zweiten Behandlung werden die Fasern wieder abgepresst und mit einer Lösung aus 55% Ethanol, 42% Wasser und 3% Zitronensäure gewaschen. Als letzter Behandlungsschritt folgt einmal Waschen mit 1% Tween® 20 in Ethanol. Danach werden die Fasern getrocknet. Die so erhaltenen Fasern haben ein Wasserrückhaltevermögen von 300% in 0,9%iger NaCl-Lösung. Da die so erhaltenen Fasern stets miteinander verklebt waren, ließ sich deren Einzelfaserfestigkeit nicht bestimmen.

## Patentansprüche

1. Wasserunlösliche Carboxyethylcellulosefasern zur Absorption von Flüssigkeiten und insbesondere Körperflüssigkeiten, beispielsweise zur Verwendung in Wundverbänden und anderen Produkten für medizinische Anwendungen oder Hygieneanwendungen und darin insbesondere zur Herstellung der Kontaktfläche zum Körper, **dadurch gekennzeichnet, dass** sie eine Festigkeit im konditionierten Zustand von mindestens 15 cN/tex und unter Beibehaltung ihrer Faserform ein Wasserrückhaltevermögen von mindestens 400 % aufweisen.

2. Carboxyethylcellulosefasern gemäß Anspruch 1, wobei die Carboxyethylcellulosefasern ein Wasserrückhaltevermögen von bevorzugt mindestens 600 %, besonders bevorzugt mindestens 800 % aufweisen.

3. Carboxyethylcellulosefasern gemäß Anspruch 1, wobei die Carboxyethylcellulosefasern eine Festigkeit im konditionierten Zustand von mindestens 20 cN/tex aufweisen.

4. Carboxyethylcellulosefasern gemäß einem der oben genannten Ansprüche, die durch Derivatisierung von Lyocell-, Viskose oder Modalfasern hergestellt wurden.

5. Verfahren zur Herstellung von wasserunlöslichen Carboxyethylcellulosefasern gemäß Anspruch 1, **dadurch gekennzeichnet, dass** cellulosische Kunstfasern mit Acrylamid in einer starken Lauge umgesetzt werden, wobei als cellulosische Kunstfasern Lyocell-, Viskose- oder Modalfasem eingesetzt werden, die Fasern nach der Umsetzung in einem nachfolgenden Schritt nochmals mit Alkalilauge nachbehandelt werden und zumindest in einem Schritt des Verfahrens die Reaktionslösung 1 bis 75 Gew.-% Ethanol enthält.

6. Verfahren gemäß Anspruch 5, wobei die Fasern in einem nachfolgenden Waschschritt mit einem Gemisch aus Ethanol, Wasser und einer schwachen Säure gewaschen werden.

7. Verfahren gemäß Anspruch 5 bis 6, wobei die cellulosischen Kunstfasern in Form von einzelnen Fasern, Endlosfaserkabel, Vliesen, textilen Flächen oder fertigen Textilien eingesetzt werden.

8. Verwendung von wasserunlöslichen Carboxyethylcellulosefasern zur Herstellung von Produkten zur Absorption von Flüssigkeiten und Körperflüssigkeiten, beispielsweise zur Verwendung in Produkten für medizinische Anwendungen oder Hygieneanwendungen, **dadurch gekennzeichnet, dass** die Carboxyethylcellulosefasern eine Festigkeit im konditionierten Zustand von mindestens 15 cN/tex und ein Wasserrückhaltevermögen von mindestens 400 % aufweisen.

9. Verwendung gemäß Anspruch 8, wobei die
Carboxyethylcellulosefasern in Form von geschnittenen Einzelfasern, Filamenten, Endlosfaserkabel, Vliesstoffen, Geweben, Gestricken, Gewirken und/oder anderen textilen Flächengebilden eingesetzt werden.

10. Verwendung gemäß Anspruch 8, wobei die
Carboxyethylcellulosefasern gemäß dem Verfahren nach Anspruch 5 bis 9 hergestellt werden.

11. Verwendung gemäß Anspruch 8 in der Kontaktfläche zum Körper.

## Claims

1. Water-insoluble carboxyethyl cellulose fibers for the absorption of liquids and particularly of body fluids, for example, for use in wound dressings and other products for medical applications or hygiene applications and therein particularly for the creation of the contact surface with the body, **characterized in that** they have a strength in the conditioned state of at least 15 cN/tex and, while maintaining their fiber form, have a water retention capacity of at least 400%.

2. The carboxyethyl cellulose fibers as claimed in claim 1, wherein the carboxylethyl cellulose fibers have a water retention capacity of preferably at least 600%, more preferably of at least 800%.

3. The carboxylethyl cellulose fibers as claimed in claim 1, wherein the carboxylethyl cellulose fibers have a strength in the conditioned state of at least 20 cN/tex.

4. The carboxyethyl cellulose fibers as claimed in any of the preceding claim, wherein said fibers are produced by derivatization of lyocell, viscose, or modal fibers.

5. A method for the production of water-insoluble carboxyethyl cellulose fibers as claimed in claim 1, **characterized in that** man-made cellulosic fibers are reacted with acrylamide in strong lye, wherein lyocell, viscose, or modal fibers are used as said man-made cellulosic fibers, the fibers are post-treated with alkaline lye in a step following the reacting step, and the reaction solution contains 1 to 75% by weight of ethanol at least in one step of the method.

6. The method as claimed in claim 5, wherein the fibers are washed in a subsequent washing step using a mixture of ethanol, water, and weak acid.

7. The method as claimed in claims 5 or 6, wherein the man-made cellulosic fibers used are in the form of individual fibers, continuous filament tows, nonwovens, textile fabrics, or finished textiles.

8. A use of water-insoluble carboxyethyl cellulose fibers for the production of products for the absorption of liquids and body fluids, for example, for use in products for medical applications or hygiene applications, **characterized in that** the carboxyethyl cellulose fibers have a strength in the conditioned state of a least 15 cN/tex and a water retention capacity of at least 400%.

9. The use as claimed in claim 8, wherein the carboxyethyl cellulose fibers used are in the form of cut individual fibers, filaments, continuous filament tows, nonwovens, woven fabrics, knitted fabrics, and/or other textile fabrics.

10. The use as claimed in claim 8, wherein the carboxyethyl cellulose fibers are produced according to the method according to claims 5 to 7.

11. The use as claimed in claim 8 in the contact surface with the body.

## Revendications

1. Fibres de carboxyéthylcellulose insolubles dans l'eau pour absorption de liquides, et notamment de liquides corporels, destinées par exemple à une utilisation dans des pansements et autres produits pour applications médicales ou hygiéniques et, dans ce cadre, destinées en particulier à la réalisation de la surface de contact avec le corps, **caractérisées en ce qu'**elles présentent une résistance d'au moins 15 cN/tex à l'état conditionné, et une capacité de rétention d'eau d'au moins 400% tout en conservant leur forme fibreuse.

2. Fibres de carboxyéthylcellulose selon la revendication 1, les fibres de carboxyéthylcellulose présentant une capacité de rétention d'eau de préférence d'au moins 600 %, ou mieux encore d'au moins 800 %.

3. Fibres de carboxyéthylcellulose selon la revendication 1, les fibres de carboxyéthylcellulose présentant une résistance d'au moins 20 cN/tex à l'état conditionné.

4. Fibres de carboxyéthylcellulose selon l'une des revendications susmentionnées, fabriquées par formation de dérivés de fibres lyocell, viscose ou modal.

5. Procédé de fabrication de fibres de carboxyéthylcellulose insolubles dans l'eau selon la revendication 1, **caractérisé en ce que** l'on fait réagir des fibres cellulosiques artificielles avec de l'acrylamide dans une lessive forte, les fibres cellulosiques artificielles utilisées étant des fibres lyocell, viscose ou modal ; **en ce que**, après la réaction, les fibres sont à nouveau post-traitées avec une lessive alcaline lors d'une étape ultérieure ; et **en ce que** lors d'une étape au moins du procédé, la solution de réaction contient 1 à 75 % d'éthanol en masse.

6. Procédé selon la revendication 5 au cours duquel les fibres sont lavées, lors d'une étape de lavage ultérieure, avec un mélange d'éthanol, d'eau et d'un acide faible.

7. Procédé selon les revendications 5 à 6, les fibres cellulosiques artificielles étant mises en oeuvre sous forme de fibres séparées, de câbles de filaments continus, de non-tissés, de surfaces textiles ou de textiles finis.

8. Utilisation de fibres de carboxyéthylcellulose insolubles dans l'eau pour la fabrication de produits pour l'absorption de liquides et de liquides corporels, par exemple pour une utilisation dans des produits destinés à des applications médicales ou hygiéniques, **caractérisée en ce que** les fibres de carboxyéthylcellulose présentent une résistance d'au moins 15 cN/tex à l'état conditionné, et une capacité de rétention d'eau d'au moins 400%.

9. Utilisation selon la revendication 8, les fibres de carboxyéthylcellulose étant mises en oeuvre sous forme de fibres séparées coupées, de filaments, de câbles de filaments continus, de non-tissés, de tissus, d'étoffes de bonneterie et/ou d'autres produits textiles.

10. Utilisation selon la revendication 8, les fibres de carboxyéthylcellulose étant fabriquées selon le procédé tel que défini dans les revendications 5 à 7.

11. Utilisation selon la revendication 8 dans la surface de contact avec le corps.
